(19) Europäisches Patentamt
European Patent Office
Office européen des brevets

(11) EP 4 372 031 A1

(12) EUROPEAN PATENT APPLICATION

(43) Date of publication:
**22.05.2024 Bulletin 2024/21**

(21) Application number: **23157569.7**

(22) Date of filing: **20.02.2023**

(51) International Patent Classification (IPC):
***C08J 3/075*** *(2006.01)* ***A61L 15/60*** *(2006.01)*
***C08L 5/08*** *(2006.01)* ***C08L 71/02*** *(2006.01)*

(52) Cooperative Patent Classification (CPC):
(C-Sets available)
**C08J 3/075; A61L 15/60; C08B 37/0072; C08G 65/3322; C08L 5/08; C08L 71/02;**
C08J 2371/02; C08J 2405/08 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.11.2022 TW 111144287**

(71) Applicant: **Win Coat Corporation**
**Hsinchu City (TW)**

(72) Inventors:
- **LIU, Ta-Jo**
  **Hsinchu City (TW)**
- **YEH, Hsiu-Feng**
  **Hsinchu City (TW)**
- **YIN, Shin-Yi**
  **Hsinchu City (TW)**
- **LIAO, Yi-Jyun**
  **Hsinchu City (TW)**
- **HSU, Ying-Hua**
  **Hsinchu City (TW)**

(74) Representative: **van Dam, Vincent**
**Octrooibureau Vriesendorp & Gaade B.V.**
**Koninginnegracht 19**
**2514 AB Den Haag (NL)**

Remarks:
Amended claims in accordance with Rule 137(2) EPC.

# (54) HYDROGEL COMPOSITION, MANUFACTURING METHOD THEREOF AND HYDROGEL MATERIAL

(57) Provided is a hydrogel composition, which comprises a modified HA, PEGDA and water; wherein, based on a total weight of the hydrogel composition, a content of the modified HA is 1 wt% to 7 wt% and a content of PEGDA is 43 wt% to 49 wt%; the modified HA is obtained by modifying HA with methacrylic anhydride; an average molecular weight of PEGDA is 1 kDa to 10 kDa. The hydrogel composition of the present invention has good compatibility of raw materials, which does not cause HA precipitation, and a hydrogel material produced therefrom simultaneously has good mechanical properties, good swell capability and good biocompatibility, such that the hydrogel material can be applied in many fields.

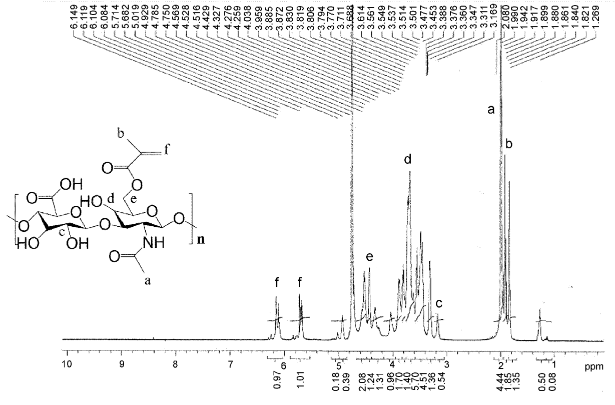

FIG.1

EP 4 372 031 A1

(52) Cooperative Patent Classification (CPC): (Cont.)

C-Sets
**C08L 71/02, C08L 5/08**

## Description

The instant disclosure relates to a composition, a manufacturing method thereof and a material, particularly to a hydrogel composition, a manufacturing method thereof and a hydrogel material.

Hydrogel, also called aqua gel, is a gel with water as dispersion medium, and has high hydrophilicity and a cross-linked structure of three-dimensional network. Due to the cross-linked structure, hydrogel will absorb and swell up rapidly in water, and it can retain a large volume of water without dissolving in this swollen state. The amount of water retained by hydrogel is closely related to the degree of crosslinking, and the higher the degree of crosslinking, the lower the amount of water retained by hydrogel. As a material of high water adsorption and high water retention, hydrogel has a wide range of applications. For example, hydrogel can be applied to daily necessities such as diapers, sanitary products or facial masks; can be applied to industrial fields such as wastewater treatment or air filtration; and can be applied to biomedical fields such as dressings for burn injury, drug delivery carriers or grafts.

In general, water-soluble or hydrophilic polymers can form hydrogel through chemical or physical crosslinking reaction. According to different sources of polymers, hydrogels are classified to natural polymer hydrogels and synthetic polymer hydrogels. The natural polymer hydrogels are formed from natural polymers, such as polysaccharides like starch, cellulose or hyaluronic acid (HA), and polypeptides like collagen or polylysine, and the synthetic polymer hydrogels are formed from synthetic polymers, such as alcohols, and acrylic acid and its derivatives like polyacrylic acid or polyacrylamide. Particularly, due to the advantages of abundant raw material sources and better biocompatibility, the application of the natural polymer hydrogel in the biomedical fields has become the focus of research in the industry and academia.

HA has excellent hydrophilicity and water retention and also widely exists in the cartilage tissue, body fluid or epidermal tissue, thereby having excellent biocompatibility. Therefore, HA is quite suitable as a raw material of natural polymer hydrogel. However, due to the high viscosity property of HA, it is difficult to inject and evenly fill the mold with HA, and thus hydrogel materials cannot be formed in specific shape to meet the different demands. Besides, HA has property of dissolving in water rapidly, and thus the hydrogel material formed by HA is unstable and easy to dissolve, and the mechanical properties thereof do not meet the actual demands, either, which limits the applicability in the biomedical fields.

For the foresaid drawbacks of HA, a technical means of mixing poly(ethylene glycol) diacrylate (PEGDA) and HA and then forming a hydrogel material has been developed. Specifically, the photo-crosslinking property of PEGDA is used to form a cross-linked structure having three-dimensional network, which can serve as the main structure for the hydrogel material and limit the degree of dissolution in water of HA, thereby improving the mechanical strength and stability of the hydrogel material. Nevertheless, in order to meet the requirements for mechanical properties in practical applications, PEGDA with higher average molecular weight, such as about 6 kilo Daltons (kDa), should be adopted as a raw material. In this circumstance, the existence of PEGDA with high average molecular weight and HA will cause an excluded volume effect, which lead to poor compatibility between the two and further make HA prone to precipitation, thereby greatly limiting the follow-up applications of the hydrogel material.

Accordingly, there is still a need to research and develop a hydrogel material adopting PEGDA and HA as raw materials, which not only makes PEGDA and HA have good compatibility to prevent HA precipitation, but also has improved mechanical properties to meet the requirements in practical applications.

In view of the problems in the prior art, an objective of the present invention is to provide a hydrogel composition comprising PEGDA and HA, and the raw materials, i.e., PEGDA and HA, have good compatibility to prevent HA precipitation, which benefits the follow-up applications when forming hydrogel material; meanwhile, a hydrogel material formed by the hydrogel composition of the present invention has improved mechanical properties, such as Young's modulus more than 450 MPa, which can meet the requirements in practical applications and thus increase the applicability.

Another objective of the present invention is to provide a hydrogel composition, and a hydrogel material formed by the hydrogel composition has good swelling capability, *i.e.,* properties of rapid water adsorption and water retention.

Still another objective of the present invention is to provide a hydrogel composition, and a hydrogel material formed by the hydrogel composition has good biocompatibility, such that the hydrogel material can be applied in the biomedical fields.

To achieve the foresaid objectives, the present invention provides a hydrogel composition, which comprises a modified HA, PEGDA and water; wherein, based on a total weight of the hydrogel composition, a content of the modified HA is 1 weight percent (wt%) to 7 wt%, and a content of PEGDA is 43 wt% to 49 wt%; the modified HA is obtained by modifying HA with methacrylic anhydride; an average molecular weight of PEGDA is 1 kDa to 10 kDa.

By adopting HA modified with methacrylic anhydride and PEGDA with higher average molecular weight as raw materials, and also controlling the contents of the modified HA and PEGD within a specific range, the hydrogel composition of the present invention does not produce HA precipitation, *i.e.*, the foresaid two raw materials have good compatibility, such that the hydrogel composition can further form a hydrogel material; meanwhile, the hydrogel material formed by the hydrogel composition of the present invention has advantages of improved mechanical properties, good swelling

capability and good biocompatibility.

In accordance with the present invention, said HA indicates a polymer of disaccharides, which are composed of N-acetyl-D-glucosamine and D-glucuronic acid, linked via β-1-3 glycosidic bonds. The molecular formula of HA is $(C_{14}H_{21}NO_{11})_n$, and the repeated unit of HA may be presented by the following Formula (a):

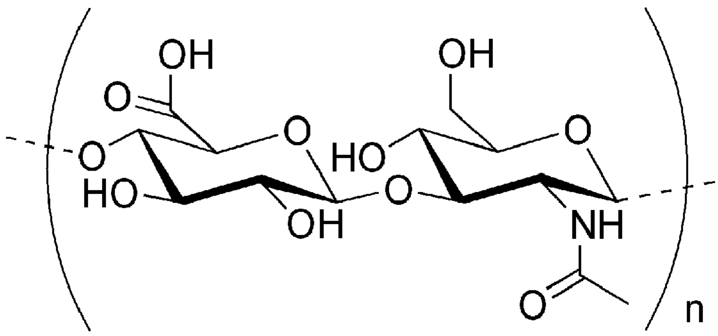

Formula (a).

In accordance with the present invention, said description "the modified HA is obtained by modifying HA with methacrylic anhydride" indicates that a methacrylate group is linked to the hydroxyl group of HA after the methacrylic anhydride reacts with HA.

In some embodiments of the present invention, when modifying HA with methacrylic anhydride to obtain the modified HA, a ratio of a mole number of said methacrylic anhydride relative to a mole number of the repeated unit of HA may be 1 to 10. In some embodiments of the present invention, a ratio of a mole number of said methacrylic anhydride relative to a mole number of the repeated unit of HA may be 1 to 4. In some embodiments of the present invention, a ratio of a mole number of said methacrylic anhydride relative to a mole number of the repeated unit of HA may be 1 to 3. In some embodiments of the present invention, a ratio of a mole number of said methacrylic anhydride relative to a mole number of the repeated unit of HA may be 2 to 3.

In some embodiments of the present invention, a ratio of methacrylate group in the modified HA may be 20% to 55%. In some embodiments of the present invention, a ratio of methacrylate group in the modified HA may be 20% to 50%. In some embodiments of the present invention, a ratio of methacrylate group in the modified HA may be 20% to 40%. Specifically, said "a ratio of methacrylate group in the modified HA" is analyzed and measured by $^1$H-nuclear magnetic resonance ($^1$H-NMR). That is, the total integral value of the measured hydrogen atoms (chemical shifts δ are about 5.56 and 6.06; and the number of hydrogen atoms is two) of methacrylate group is divided with the total integral value of the measured hydrogen atoms (chemical shifts δ are about 3.0 to 4.5; and the number of hydrogen atoms is ten) of the backbone of HA, so as to obtain said "a ratio of methacrylate group in the modified HA".

In some embodiments of the present invention, based on the total weight of the hydrogel composition, the content of the modified HA may be 2.5 wt% to 7 wt%. In some embodiments of the present invention, based on the total weight of the hydrogel composition, the content of the modified HA may be 2.5 wt% to 5 wt%. In some embodiments of the present invention, based on the total weight of the hydrogel composition, the content of the modified HA may be 4 wt% to 6 wt%.

In some embodiments of the present invention, based on the total weight of the hydrogel composition, the content of PEGDA may be 43 wt% to 47.5 wt%. In some embodiments of the present invention, based on the total weight of the hydrogel composition, the content of PEGDA may be 45 wt% to 47.5 wt%. In some embodiments of the present invention, based on the total weight of the hydrogel composition, the content of PEGDA may be 44 wt% to 46 wt%.

In some embodiments of the present invention, based on the total weight of the hydrogel composition, a content of water may be 40 wt% to 60 wt%. In some embodiments of the present invention, based on the total weight of the hydrogel composition, a content of water may be 44 wt% to 56 wt%.

In some embodiments of the present invention, based on the total weight of the hydrogel composition, the content of the modified HA may be 2.5 wt% to 5 wt%, the content of PEGDA may be 45 wt% to 47.5 wt%, and the ratio of the mole number of said methacrylic anhydride relative to the mole number of the repeated unit of HA may be 1 to 3.

In some embodiments of the present invention, based on the total weight of the hydrogel composition, the content of the modified HA may be 4 wt% to 6 wt%, the content of PEGDA may be 44 wt% to 46 wt%, and the ratio of the mole number of said methacrylic anhydride relative to the mole number of the repeated unit of HA may be 1 to 3.

In some embodiments of the present invention, based on the total weight of the hydrogel composition, the content of the modified HA may be 4 wt% to 6 wt%, the content of PEGDA may be 44 wt% to 46 wt%, and the ratio of the mole number of said methacrylic anhydride relative to the mole number of the repeated unit of HA may be 2 to 3.

In some embodiments of the present invention, the average molecular weight of PEGDA may be 4 kDa to 8

kDa. In some embodiments of the present invention, the average molecular weight of PEGDA may be 5 kDa to 7 kDa.

In some embodiments of the present invention, an average molecular weight of HA may be 1 kDa to 200 kDa. In some embodiments of the present invention, an average molecular weight of HA may be 1 kDa to 100 kDa.

Preferably, the hydrogel composition may further comprise a photoinitiator. The photoinitiator may be a compound which initiates monomer polymerization, crosslinking and curing after light exposure. For example, the photoinitiator may be, but is not limited to, lithium phenyl-2,4,6-trimethylbenzoylphosphinate (LAP), 2,4,6-trimethylbenzoyl diphenylphosphine oxide (also named as Irgacure TPO), 2-Hydroxy-4'-(2-hydroxyethoxy)-2-methylpropiophenone (also named as Irgacure 2959), or Eosin Y. More preferably, the photoinitiator may be LAP.

In some embodiments of the present invention, based on the total weight of the hydrogel composition, a content of the photoinitiator may be 0.2 wt% to 0.8 wt%. In some embodiments of the present invention, based on the total weight of the hydrogel composition, a content of the photoinitiator may be 0.4 wt% to 0.6 wt%.

Besides, the present invention also provides a manufacturing method of a hydrogel composition, which comprises the following steps: step (a): modifying HA with methacrylic anhydride to obtain a modified HA; and step (b): mixing the modified HA, PEGDA and water to obtain the hydrogel composition; wherein, based on a total weight of the hydrogel composition, a content of the modified HA is 1 wt% to 7 wt%, and a content of PEGDA is 43 wt% to 49 wt%; an average molecular weight of PEGDA is 1 kDa to 10 kDa.

By modifying HA with methacrylic anhydride to obtain the modified HA in advance, and then mixing the modified HA and PEGDA with higher average molecular weight as raw materials, and also controlling the contents of the modified HA and PEGD within a specific range, the prepared hydrogel composition does not produce HA precipitation, *i.e.*, the raw materials have good compatibility, such that the hydrogel composition can further form a hydrogel material; meanwhile, the hydrogel material has advantages of improved mechanical properties, good swelling capability and good biocompatibility.

In some embodiments of the present invention, in the step (a), a ratio of a mole number of said methacrylic anhydride relative to a mole number of the repeated unit of HA may be 1 to 10; in some embodiments of the present invention, a ratio of a mole number of said methacrylic anhydride relative to a mole number of the repeated unit of HA may be 1 to 4; in some embodiments of the present invention, a ratio of a mole number of said methacrylic anhydride relative to a mole number of the repeated unit of HA may be 1 to 3; and in some embodiments of the present invention, a ratio of a mole number of said methacrylic anhydride relative to a mole number of the repeated unit of HA may be 2 to 3.

Preferably, in the step (a), when modifying HA with methacrylic anhydride, a reaction time may be 20 hours to 30 hours, and a reaction temperature may be 0°C to 8°C. More preferably, in the step (a), when modifying HA with methacrylic anhydride, a reaction time may be 20 hours to 30 hours, and a reaction temperature may be 0°C to 5°C.

Besides, the present invention also provides a hydrogel material, which is formed from the foresaid hydrogel composition of the present invention. The hydrogel material of the present invention has advantages of improved mechanical properties, good swelling capability and good biocompatibility, such that the hydrogel material can be widely applied in various fields, especially the biomedical fields.

Preferably, the hydrogel material has Young's modulus more than 450 MPa. More preferably, the hydrogel material has Young's modulus more than 450 MPa and less than or equal to 700 MPa. Even more preferably, the hydrogel material has Young's modulus more than or equal to 480 MPa and less than or equal to 700 MPa.

Preferably, an ultimate tensile strength (UTS) of the hydrogel material is more than 13 MPa. More preferably, an ultimate tensile strength of the hydrogel material is more than 13 MPa and less than or equal to 18 MPa. Even more preferably, an ultimate tensile strength of the hydrogel material is more than or equal to 15 MPa and less than or equal to 18 MPa. Even more preferably, an ultimate tensile strength of the hydrogel material is more than or equal to 17 MPa and less than or equal to 18 MPa.

In the specification, a range represented by "a lower-endpoint value to an upper-endpoint value", if not particularly specified, indicates that the range is more than or equal to the lower-endpoint value and less than or equal to the upper-endpoint value. For example, "a content is 1 wt% to 7 wt%" indicates that a content is "more than or equal to 1 wt% and less than or equal to 7 wt%".

Other objectives, advantages and novel features of the instant disclosure will become more apparent from the following detailed description when taken in conjunction with the accompanying drawings.

In the drawings:

FIG. 1 is $^1$H-NMR result of the modified HA of Preparation Example 4.
FIG. 2 is the results of cell viability test of the hydrogel material of Example 3A.

Hereinafter, several preparation methods of embodiments are exemplified to illustrate the implementation of the present invention. One person skilled in the art can easily realize the advantages and effects of the present invention in accordance with the contents of the specification.

**Description of Reagents**

1. HA: purchased from Kewpie Corporation; the average molecular weight is about 7 kDa.
2. PEGDA: purchased from Sigma-Aldrich; the average molecular weight is about 6 kDa.
3. Methacrylic anhydride: purchased from Sigma-Aldrich.
4. LAP: purchased from Sigma-Aldrich.
5. N,N-dimethylacetamid (DMAC): purchased from Duksan Pure Chemicals Co., Ltd.

**Preparation Examples 1 to 4 (PE1 to PE4) : Modified HA**

0.5 mole of HA and about 10 milliliters (mL) of deionized water were added into a two-neck round bottle flask, and then placed at 4°C environment for overnight, so as to completely dissolve HA in deionized water. Next, about 6.7 mL of DMAC was added into the two-neck round bottle flask. Afterward, according to the different ratios between the mole number of methacrylic anhydride and the mole number of the repeated unit of HA listed in the following Table 1, the suitable amount of methacrylic anhydride was added into the two-neck round bottle flask, and then placed in ice bath, followed by mixing uniformly the solution therein. Next, 0.5 molarity (M) of NaOH was added into the mixed solution for titration, and the pH value of the mixed solution was controlled within about 8 to 9 for 4 hours. Then, the mixed solution was placed at 4°C environment and then continuously stirred for about 20 hours of reaction.

After the reaction was completed, the suitable amount of sodium chloride was added into the solution to make the concentration thereof equal to 0.5 M. Next, the solution was mixed with ethanol under volume proportion of 1:9 to produce precipitate. Then, after the precipitate was rinsed with ethanol to remove unreacted matters, the precipitate was dissolved in deionized water to obtain a crude product. Next, the crude product was purified with dialysis in deionized water for 3 days, followed by lyophilization to obtain the modified HA of Preparation Examples 1 to 4.

Table 1: The ratios between the mole number of methacrylic anhydride and the mole number of the repeated unit of HA adopted by the modified HA of PE 1 to PE 4.

| **Groups** | **Mole number of methacrylic anhydride: Mole number of the repeated unit of HA** |
|---|---|
| PE 1 | 1 : 1 |
| PE 2 | 3 : 1 |
| PE 3 | 4 : 1 |
| PE 4 | 10 : 1 |

Besides, in order to verify that methacrylate group was contained in the modified HA of PE 1 to PE 4, the modified HA of PE 4 was analyzed with $^1$H-NMR spectrometer (manufacturer: Varian, Inc.; model: VNMRS 700 MHz) as an example for explanation. The $^1$H-NMR result of the modified HA of PE 4 was shown in FIG.1, and the adopted d-solvent was $D_2O$. According to FIG.1, the obvious peaks of chemical shift at about 1.86 ppm, *i.e.*, mark b in FIG.1, and 5.56 ppm and 6.06 ppm, *i.e.*, mark f in FIG.1, can verify that methacrylate group was actually linked to the structure of HA, and the chemical structure of the modified HA was represented by the following Formula (b). Besides, by calculating a ratio between a total integral value of hydrogen atoms of methacrylate group and a total integral value of hydrogen atoms of the backbone of HA, the result that a ratio of methacrylate group in the modified HA of PE 4 was about 54.1% could be obtained.

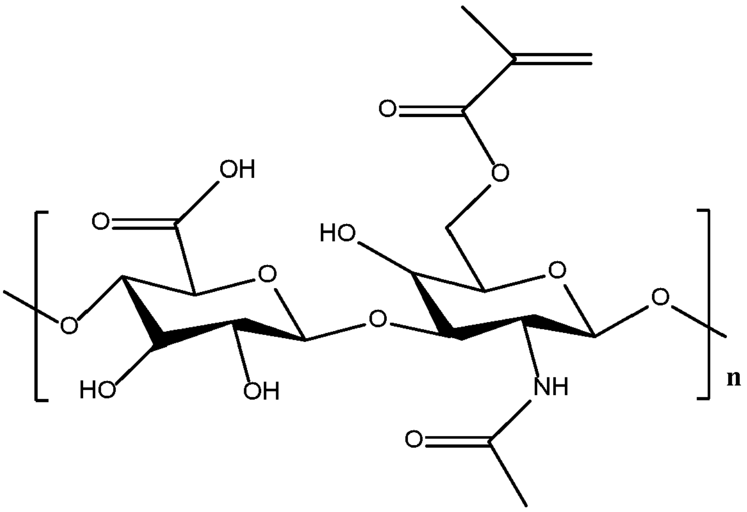

Formula (b).

**Examples 1 to 5 (E1 to E5): Hydrogel composition**

According to the composition and the content listed in the following Table 2, the suitable amount of the modified HA of PE1 to PE 4, PEGDA and deionized water were provided and then mixed uniformly to obtain the hydrogel compositions of Examples 1 to 5.

**Comparative Example 1 (CE1): PEGDA solution**

According to the following Table 2, the suitable amount of PEGDA and deionized water were mixed uniformly to obtain the PEGDA solution of Comparative Example 1; wherein, based on a total weight of the PEGDA solution, a content of PEGDA was 50 wt% and the rest was deionized water.

**Comparative Example 2 (CE2): HA/PEGDA solution**

According to the following Table 2, the suitable amount of HA (unmodified HA), PEGDA and deionized water were mixed uniformly to obtain the HA/PEGDA solution of Comparative Example 2; wherein, based on a total weight of the HA/PEGDA solution, a content of HA was 5 wt%, a content of PEGDA was 45 wt% and the rest was deionized water.

**Comparative Examples 3 and 4 (CE3 and CE4): Hydrogel composition**

According to the composition and the content listed in the following Table 2, the suitable amount of the modified HA of PE2, PEGDA and deionized water were provided and then mixed uniformly to obtain the hydrogel compositions of Comparative Examples 3 and 4. The main difference between the hydrogel compositions of Comparative Examples 3 and 4 and the hydrogel compositions of Examples 1 to 5 were that the contents of the modified HA and PEGDA were different.

Table 2: The compositions and contents of the hydrogel compositions of E1 to E5, the PEGDA solution of CE1, the HA/PEGDA solution of CE2 and the hydrogel compositions of CE3 and CE4.

| **Groups** | **HA** | | **PEGDA (wt%)** | **Deionized water (wt%)** |
|---|---|---|---|---|
| | **Type** | **Content (wt%)** | | |
| E1 | Modified HA of PE2 | 2.5 | 47.5 | 50 |
| E2 | Modified HA of PE1 | 5 | 45 | 50 |
| E3 | Modified HA of PE2 | 5 | 45 | 50 |
| E4 | Modified HA of PE3 | 5 | 45 | 50 |
| E5 | Modified HA of PE4 | 5 | 45 | 50 |
| CE1 | -- | -- | 50 | 50 |

(continued)

| Groups | HA | | PEGDA (wt%) | Deionized water (wt%) |
|---|---|---|---|---|
| | Type | Content (wt%) | | |
| CE2 | Unmodified HA | 5 | 45 | 50 |
| CE3 | Modified HA of PE2 | 10 | 40 | 50 |
| CE4 | Modified HA of PE2 | 15 | 35 | 50 |

**Analysis 1: Evaluation of compatibility of raw materials**

The hydrogel compositions of E2 to E5 and the HA/PEGDA solution of CE2 were adopted for this analysis. Specifically, the same amount of the hydrogel compositions of E2 to E5 and the HA/PEGDA solution of CE2 were adopted and respectively placed into different containers with the same condition.

Next, the clarity of each group was observed with naked eyes, and obvious precipitates, *i.e.*, HA precipitation, were observed in the HA/PEGDA solution of CE2. That is, the HA/PEGDA solution of CE2 obviously had poor compatibility of raw materials. On the other hand, no obvious precipitates were observed in the hydrogel compositions of E2 to E5, which indicated that all of the hydrogel compositions of E2 to E5 had good compatibility of raw materials. Besides, the hydrogel composition of E5 had the best clarity, and thus had the best compatibility of raw materials.

Accordingly, unlike the problem existed in the prior art that directly mixing HA and PEGDA caused HA precipitation, the hydrogel composition of the present invention adopted HA modified with methacrylic anhydride and then mixed with PEGDA, such that the compatibility of raw materials was enhanced and the problem of HA precipitating in PEGDA was solved.

**Examples 1A to 5A (E1A to E5A): Hydrogel material**

Examples 1A to 5A adopted the hydrogel compositions of E1 to E5, respectively, and then the suitable amount of LAP was added to reach the content about 0.5 wt% as a photoinitiator. Next, the hydrogel composition of each group was placed into a mold, and then exposed to ultraviolet light (the light source was an LED with a wavelength of 390 nanometers (nm), and the light power was about 5 watts) for about 30 seconds to make the hydrogel composition undergo crosslinking reaction and then curing to obtain the hydrogel materials of Examples 1A to 5A.

**Comparative Examples 1A to 4A (CE1A to CE4A): Hydrogel material**

The manufacturing processes of Comparative Examples 1A to 4A were similar to those of E1A to E5A. The main difference was that Comparative Examples 1A to 4A respectively adopted the PEGDA solution of CE1, the HA/PEGDA solution of CE2 and the hydrogel compositions of CE3 and CE4. Afterward, the manufacturing processes were the same as those of E1A to E5A to obtain the hydrogel materials of Comparative Examples 1A to 4A.

**Analysis 2: Evaluation of mechanical properties**

The hydrogel materials of E1A to E5A and CE1A to CE4A were adopted for this analysis. Specifically, the hydrogel materials of E1A to E5A and CE1A to CE4A were cut into samples with length of about 30 millimeters (mm), width of about 3 mm and height of about 0.5 mm. Next, each sample was placed onto ElectroForce® 3200 Series III mechanical property tester, and the conditions of 225 Newton (N) load cell and extending rate of 0.08 millimeters/minute (mm/min) were selected for the test, and then the results of Young's modulus and ultimate tensile strength of each group were obtained and listed in the following Table 3. Young's modulus was obtained by calculating the slope of the linear elastic part of the stress-strain curve, which indicated the difficulty of deformation of an object after giving a force. Briefly, the higher the Young's modulus was, the more difficult the object would deform after subjected to a force. Ultimate tensile strength was the measured force when the object was stretched until breakage.

Table 3: The results of Young's modulus and ultimate tensile strength of the hydrogel materials of E1A to E5A and CE1A to CE4A.

| Groups | Young's modulus (MPa) | Ultimate tensile strength (MPa) |
|---|---|---|
| E1A | 485.56 | 17.7 |

(continued)

| Groups | Young's modulus (MPa) | Ultimate tensile strength (MPa) |
|---|---|---|
| E2A | 567.32 | 15.28 |
| E3A | 691.36 | 17.2 |
| E4A | 454.7 | 13.29 |
| E5A | 452.1 | 13.81 |
| CE1A | 378.79 | 15.41 |
| CE2A | 426.61 | 12.43 |
| CE3A | 316.8 | 9.66 |
| CE4A | 74.38 | 5.42 |

According to the results shown in Table 3 above, Young's modulus of the hydrogel materials of E1A to E5A were more than 450 MPa. Besides, when compared to the hydrogel material of CE1A, which was formed from the PEGDA solution or the hydrogel material of CE2A, which was formed from the HA/PEGDA solution, the hydrogel materials of E1A to E5A had obviously higher Young's modulus. That is, as the present invention adopted HA modified with methacrylic anhydride and then mixed with PEGDA to form the hydrogel material, the hydrogel material actually had better mechanical strength.

Further compared to the results of CE3A and CE4A, although the hydrogel materials of CE3A and CE4A were also adopted HA modified with methacrylic anhydride and then mixed with PEGDA, for the hydrogel material of CE3A, the content of the modified HA was 10 wt%, and the content of PEGDA was 40 wt%; and for the hydrogel material of CE4A, the content of the modified HA was 15 wt%, and the content of PEGDA was 35 wt%. That is, the contents of the raw materials of CE3A and CE4A did not meet the specific range as claimed by the present invention. Hence, Young's modulus of the hydrogel material of CE3A was only 378.79 MPa, and Young's modulus of the hydrogel material of CE4A was only 74.39 MPa, which was obviously worse than those of the hydrogel materials of E1A to E5A.

Besides, for the results of ultimate tensile strength, the hydrogel materials of E1A to E5A were all more than 13 MPa, and the hydrogel materials of E1A to E5A had close or even higher ultimate tensile strength than CE1A. Also, when compared to CE2A to CE4A, the hydrogel materials of E1A to E5A had obviously higher ultimate tensile strength.

Accordingly, as the present invention adopted HA modified with methacrylic anhydride and PEGDA as raw materials, and also controlled the contents of the raw materials within a specific range, the hydrogel material of the present invention actually had better result of Young's modulus and close or event better result of ultimate tensile strength. That is, the hydrogel material of the present invention actually had improved mechanical properties.

**Analysis 3: Evaluation of swelling capability**

The hydrogel materials of E1A to E5A were adopted for this analysis. Specifically, the hydrogel materials of E1A to E5A were cut into cylindrical samples with diameter of about 15 mm and thickness of about 0.5 mm. Next, the samples were soaked in 5 mL deionized water, and then the weights of samples were measured when the samples were soaked in deionized water for 30 seconds, 3 minutes, 5 minutes, 10 minutes, 15 minutes, 20 minutes, 30 minutes and 60 minutes. Afterward, the measured sample weight based on different soaking time was compared to the sample weight before soaking in water, and the swelling rate of each group after soaking in water for different timespans was obtained, and then used to evaluate swelling capability of the hydrogel material of each group. The results of swelling rate after soaking in water for different timespan of different groups were listed in the following Table 4. The swelling rate was obtained by the following formula: [(sample weight after soaking in water - sample weight before soaking in water) / sample weight before soaking in water$\times$100%].

Table 4: The results of swelling rate of the hydrogel materials of E1A to E5A after soaking in water for different timespans.

| Groups | Swelling rate after soaking in water for different timespans (%) | | | | | | | | |
|---|---|---|---|---|---|---|---|---|---|
| | 0. 5 mi n | 1 min | 3 min | 5 min | 10 min | 15 min | 20 min | 30 min | 60 min |
| E1A | 47 | 109 | 249 | 318 | 353 | 354 | 353 | 348 | 340 |
| E2A | 74 | 164 | 286 | 325 | 329 | 325 | 323 | 320 | 313 |

(continued)

| Groups | Swelling rate after soaking in water for different timespans (%) | | | | | | | | |
|---|---|---|---|---|---|---|---|---|---|
| | 0. 5 mi n | 1 min | 3 min | 5 min | 10 min | 15 min | 20 min | 30 min | 60 min |
| E3A | 60 | 142 | 231 | 240 | 238 | 236 | 234 | 229 | 226 |
| E4A | 50 | 124 | 204 | 210 | 209 | 206 | 202 | 201 | 196 |
| E5A | 42 | 102 | 198 | 212 | 210 | 206 | 205 | 204 | 199 |

According to the results shown in above Table 4, the hydrogel materials of E1A to E5A all had the swelling rate more than 200% after soaking in water for about 5 minutes. Besides, when soaking in water for longer timespans (about 10 minutes to 60 minutes), the hydrogel materials of E1A to E5A still maintained the swelling rates more than 200%, and the swelling rates thereof almost were a fixed value, which indicated that the hydrogel materials of E1A to E5A had the properties of rapid water adsorption and long water retention. Accordingly, the hydrogel material of the present invention actually had good swelling capability.

**Analysis 4: Evaluation of biocompatibility**

The hydrogel material of E3A was adopted for this analysis, and a reagent of PrestoBlue was used to test the cell viability, so as to evaluate the biocompatibility. Specifically, the hydrogel material of E3A was soaked in deionized water to make its swelling rate reach a fixed value, and then was cut into cylindrical sample with diameter of about 6 mm and thickness of about 0.5 mm. Next, the sample was soaked into ethanol with concentration of 30%, and then placed under an environment with ultraviolet light for sterilization. Afterward, the sample was taken out and washed with phosphate buffered solution and then placed into a 96-well cell culture plate, followed by adding 200 microliters ($\mu$L) of Ham's F12 culture medium. At the same time, HIG-82 cells (cells isolated from synovium of a knee joint of a rabbit) with cell concentration of about $5\times10^4$ cell/mL were placed in another 96-well cell culture plate, and then cultured in an environment of about 5% $CO_2$ at about 37°C for 1 day. Then, the sample soaked with Ham's F12 culture medium was taken out, and then added into the 96-well cell culture plate cultured with HIG-82 cells, and then cultured for 1 day.

After the foresaid culture completed, Ham's F12 culture medium was removed from the 96-well cell culture plate, and then 100 $\mu$L of PrestoBlue solution (PrestoBlue reagent mixed with Ham's F12 culture medium at a volume ratio of 1:9) was added, and then cultured in an environment of about 5% $CO_2$ at about 37°C for 1.5 hours. Next, the PrestoBlue solution was taken out and analyzed with enzyme-linked immunosorbent assay (ELISA) reader (manufacturer: Molecular Devices; model: SpectraMax M2) to measure the absorbance at wavelengths of 570 nm and 600 nm for calculating the reduction of PrestoBlue, and the higher the reduction of PrestoBlue, the higher the cell viability, and also the better the biocompatibility of the sample. The results of E3A were shown as FIG.2; wherein, HIG-82 cells without any treatment were set as the control group, and each group was the results of 5 repeated experiments. The reduction of PrestoBlue was obtained by the following formula; wherein, in the formula, the test group represented the foresaid group treated with the sample and the control group, and the negative group represented the group that went through the same experimental processes mentioned above but did not contain HIG-82 cells.

$$\text{Reduction of PrestoBkue (\%)} = \frac{C_{RED}\text{ of test group}}{C_{OX}\text{ of negative group}} \times 100\%$$
$$= \frac{(\varepsilon_{OX}\lambda_{600})(A\lambda_{570}) - (\varepsilon_{OX}\lambda_{570})(A\lambda_{600})}{(\varepsilon_{RED}\lambda_{570})(A'\lambda_{600}) - (\varepsilon_{RED}\lambda_{600})(A'\lambda_{570})} \times 100\%.$$

$C_{RED}$: concentration of reduced form PrestoBlue (red). $C_{OX}$: concentration of oxidized form PrestoBlue (blue).
$\varepsilon_{OX}\lambda_{570}$: molar extinction coefficient of oxidized form PrestoBlue at wavelength of 570 nm.
$\varepsilon_{OX}\lambda_{600}$: molar extinction coefficient of oxidized form PrestoBlue at wavelength of 600 nm.
$\varepsilon_{RED}\lambda_{570}$: molar extinction coefficient of reduced form PrestoBlue at wavelength of 570 nm.
$\varepsilon_{RED}\lambda_{600}$: molar extinction coefficient of reduced form PrestoBlue at wavelength of 600 nm.
A: absorbance of test groups (contained culture medium, the sample, the cells and PrestoBlue reagent).
A': absorbance of negative control (contained culture medium, the sample and PrestoBlue reagent).

According to FIG.2, the reduction of PrestoBlue of E3A and the control group were almost the same, which indicated that the cell viability of E3A and the control group were almost the same. That is, the hydrogel material of E3A

did not release substances which were toxic to cell, infectious, or stimulated cell to inflammation and thus cause cell death. Therefore, the hydrogel material of the present invention actually had good biocompatibility.

In summary, as the present invention adopts the modified HA and PEGDA with higher average molecular weight as raw materials, and also controlled the contents of the raw materials, a hydrogel composition with good compatibility of raw materials can be obtained. Meanwhile, after the hydrogel composition forms a hydrogel material, the hydrogel material simultaneously has good mechanical properties, good swelling capability and good biocompatibility, such that the hydrogel material can be applied in numerous fields, such as the biomedical fields, thereby having high developmental potential and value.

## Claims

1. A hydrogel composition, **characterized by** comprising a modified hyaluronic acid, poly(ethylene glycol) diacrylate and water; wherein, based on a total weight of the hydrogel composition, a content of the modified hyaluronic acid is 1 weight percent to 7 weight percent, and a content of poly(ethylene glycol) diacrylate is 43 weight percent to 49 weight percent; the modified hyaluronic acid is obtained by modifying hyaluronic acid with methacrylic anhydride; an average molecular weight of poly(ethylene glycol) diacrylate is 1 kDa to 10 kDa.

2. The hydrogel composition as claimed in claim 1, **characterized in that** a ratio of a mole number of methacrylic anhydride relative to a mole number of a repeated unit of hyaluronic acid is 1 to 10.

3. The hydrogel composition as claimed in claims 1 or 2, **characterized in that** the average molecular weight of poly(ethylene glycol) diacrylate is 4 kDa to 8 kDa.

4. The hydrogel composition as claimed in any one of claims 1 to 3, **characterized in that** an average molecular weight of hyaluronic acid is 1 kDa to 200 kDa.

5. The hydrogel composition as claimed in any one of claims 1 to 4, **characterized in that** the hydrogel composition further comprises a photoinitiator.

6. A manufacturing method of a hydrogel composition, **characterized by** comprising the following steps:

   step (a): modifying hyaluronic acid with methacrylic anhydride to obtain a modified hyaluronic acid; and
   step (b): mixing the modified hyaluronic acid, poly(ethylene glycol) diacrylate and water to obtain the hydrogel composition;
   wherein, based on a total weight of the hydrogel composition, a content of the modified hyaluronic acid is 1 weight percent to 7 weight percent, and a content of poly(ethylene glycol) diacrylate is 43 weight percent to 49 weight percent; an average molecular weight of poly(ethylene glycol) diacrylate is 1 kDa to 10 kDa.

7. The manufacturing method as claimed in claim 6, **characterized in that** a ratio of a mole number of methacrylic anhydride relative to a mole number of a repeated unit of hyaluronic acid is 1 to 10.

8. The manufacturing method as claimed in claims 6 or 7, **characterized in that** in the step (a), when modifying hyaluronic acid with methacrylic anhydride, a reaction time is 20 hours to 30 hours, and a reaction temperature is 0°C to 8°C.

9. A hydrogel material, **characterized in that** the hydrogel material is formed from the hydrogel composition as claimed in any one of claims 1 to 5.

10. The hydrogel material as claimed in claim 9, **characterized in that** Young's modulus of the hydrogel material is more than 450 MPa.

## Amended claims in accordance with Rule 137(2) EPC.

1. A hydrogel composition, **characterized by** comprising a modified hyaluronic acid, poly(ethylene glycol) diacrylate and water; wherein, based on a total weight of the hydrogel composition, a content of the modified hyaluronic acid is 1 weight percent to 7 weight percent, and a content of poly(ethylene glycol) diacrylate is 43 weight percent to 49

weight percent; the modified hyaluronic acid is obtained by modifying hyaluronic acid with methacrylic anhydride; an average molecular weight of poly(ethylene glycol) diacrylate is 1 kDa to 10 kDa.

**2.** The hydrogel composition as claimed in claim 1, **characterized in that** a ratio of a mole number of methacrylic anhydride relative to a mole number of a repeated unit of hyaluronic acid is 1 to 10.

**3.** The hydrogel composition as claimed in claim 1 or 2, **characterized in that** the average molecular weight of poly(ethylene glycol) diacrylate is 4 kDa to 8 kDa.

**4.** The hydrogel composition as claimed in any one of claims 1 to 3, **characterized in that** an average molecular weight of hyaluronic acid is 1 kDa to 200 kDa.

**5.** The hydrogel composition as claimed in any one of claims 1 to 4, **characterized in that** the hydrogel composition further comprises a photoinitiator.

**6.** A manufacturing method of a hydrogel composition, **characterized by** comprising the following steps:

step (a): modifying hyaluronic acid with methacrylic anhydride to obtain a modified hyaluronic acid; and
step (b): mixing the modified hyaluronic acid, poly(ethylene glycol) diacrylate and water to obtain the hydrogel composition;

wherein, based on a total weight of the hydrogel composition, a content of the modified hyaluronic acid is 1 weight percent to 7 weight percent, and a content of poly(ethylene glycol) diacrylate is 43 weight percent to 49 weight percent; an average molecular weight of poly(ethylene glycol) diacrylate is 1 kDa to 10 kDa.

**7.** The manufacturing method as claimed in claim 6, **characterized in that** a ratio of a mole number of methacrylic anhydride relative to a mole number of a repeated unit of hyaluronic acid is 1 to 10.

**8.** The manufacturing method as claimed in claim 6 or 7, **characterized in that** in the step (a), when modifying hyaluronic acid with methacrylic anhydride, a reaction time is 20 hours to 30 hours, and a reaction temperature is 0°C to 8°C.

**9.** A hydrogel material, **characterized in that** the hydrogel material is formed from the hydrogel composition as claimed in any one of claims 1 to 5.

**10.** The hydrogel material as claimed in claim 9, **characterized in that** Young's modulus of the hydrogel material is more than 450 MPa; wherein, Young's modulus is measured in conditions using a 225 Newtons load cell and an extending rate of 0.08 millimeters/minute.

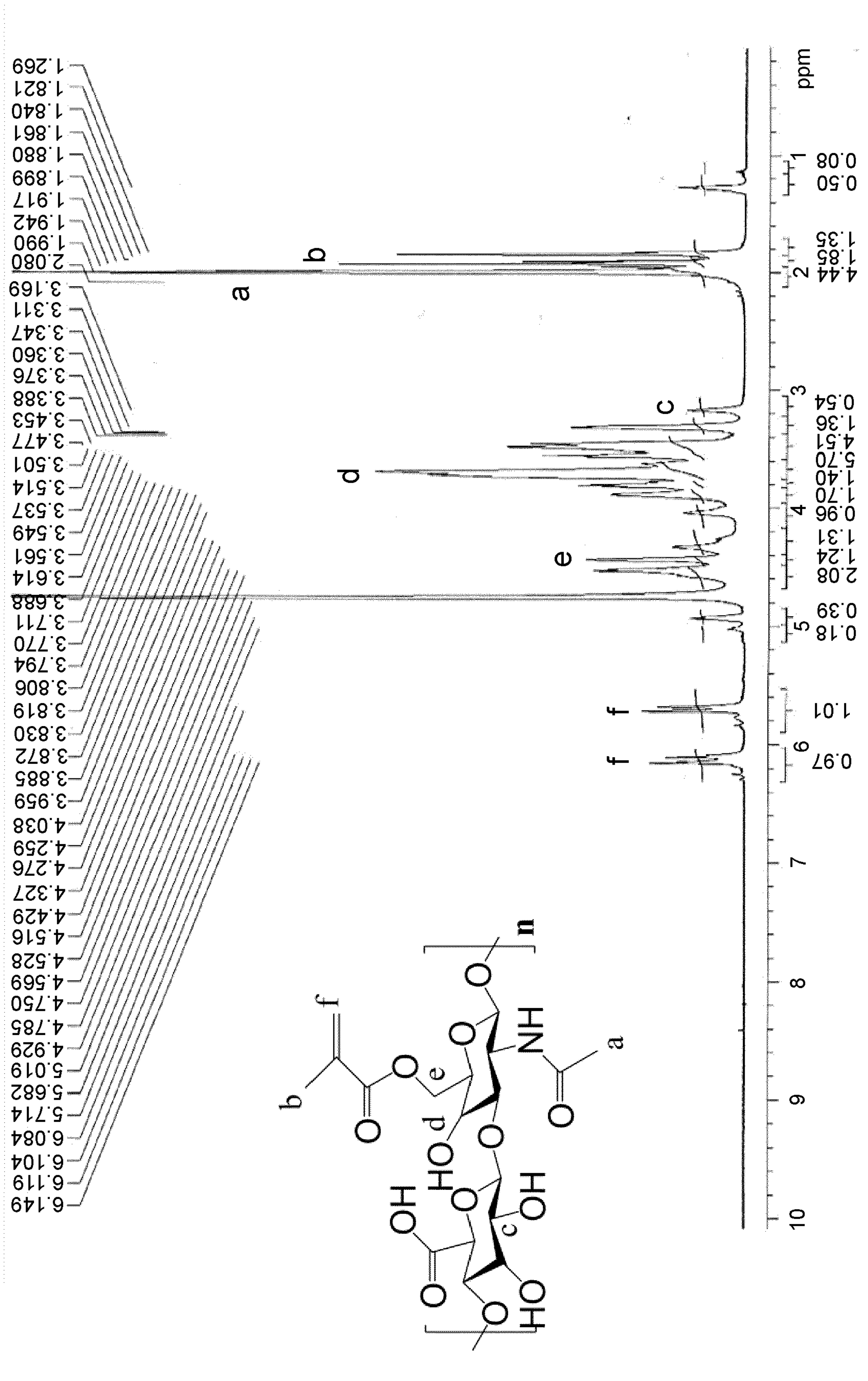
1.269
1.821
1.840
1.861
1.880
1.899
1.917
1.942
1.990
2.080
3.169
3.311
3.347
3.360
3.376
3.388
3.453
3.477
3.501
3.514
3.537
3.549
3.561
3.614
3.688
3.711
3.770
3.794
3.806
3.819
3.830
3.872
3.885
3.959
4.038
4.259
4.276
4.327
4.429
4.516
4.528
4.569
4.750
4.785
4.929
5.019
5.682
5.714
6.084
6.104
6.119
6.149
a
b
c
d
e
f
f
0.08
0.50
1.35
1.85
4.44
0.54
1.36
4.51
5.70
1.40
1.70
0.96
1.31
1.24
2.08
0.39
0.18
1.01
0.97
ppm

FIG.1

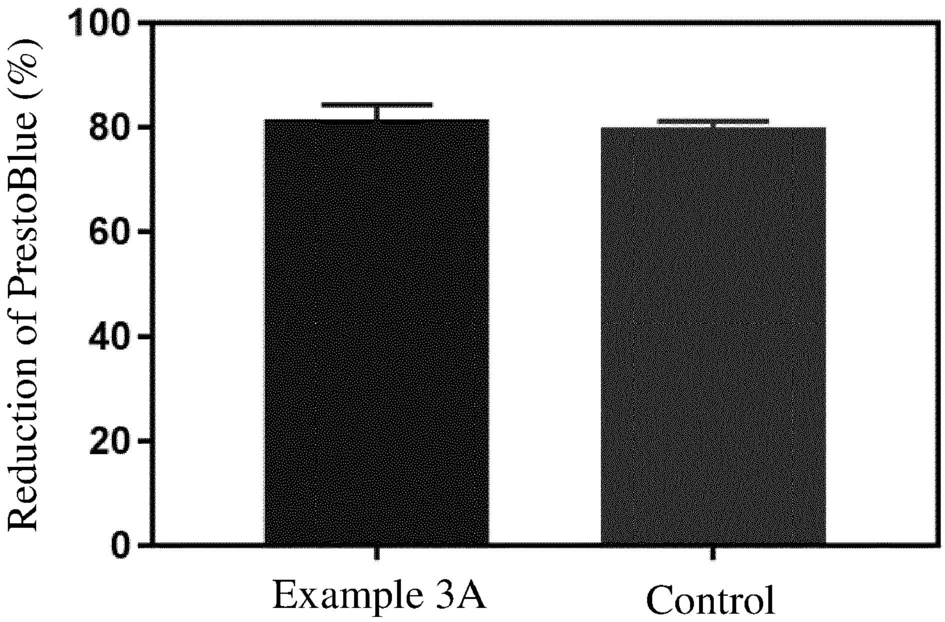


FIG.2

Europäisches Patentamt
European Patent Office
Office européen des brevets

# EUROPEAN SEARCH REPORT

Application Number
EP 23 15 7569

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | D'AMORA UGO ET AL: "Bioactive composites based on double network approach with tailored mechanical, physico-chemical, and biological features : Bioactive composites based on double network approach", JOURNAL OF BIOMEDICAL MATERIALS RESEARCH PART A, [Online] vol. 106, no. 12, 12 September 2018 (2018-09-12), pages 3079-3089, XP093069729, US ISSN: 1549-3296, DOI: 10.1002/jbm.a.36498 Retrieved from the Internet: URL:https://api.wiley.com/onlinelibrary/tdm/v1/articles/10.1002%2Fjbm.a.36498> [retrieved on 2023-08-02] * abstract * * "Experimental"; page 3080 - page 3081 * * figure 8; tables I, II * ----- | 1-10 | INV. C08J3/075 A61L15/60 C08L5/08 C08L71/02 |
| A | CN 106 474 049 A (UNIV EAST CHINA NORMAL) 8 March 2017 (2017-03-08) * WPI abstract * * claims 1-3 * ----- | 1-10 | TECHNICAL FIELDS SEARCHED (IPC) C08J A61L C09J C08L |
| A | CN 114 146 226 A (UNIV ZHEJIANG) 8 March 2022 (2022-03-08) * WPI abstract * * claims 1-10 * ----- -/-- | 1-10 | |

The present search report has been drawn up for all claims

1

| Place of search | Date of completion of the search | Examiner |
|---|---|---|
| Munich | 3 August 2023 | Meiser, Wibke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 23 15 7569

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHOI JANE RU ET AL: "Recent advances in photo-crosslinkable hydrogels for biomedical applications", BIOTECHNIQUES, [Online] vol. 66, no. 1, 1 January 2019 (2019-01-01), pages 40-53, XP093006521, US ISSN: 0736-6205, DOI: 10.2144/btn-2018-0083 Retrieved from the Internet: URL:https://www.future-science.com/doi/10.2144/btn-2018-0083> [retrieved on 2022-12-09] * page 50, right-hand column * ----- | 1-10 | |
| A | CA 3 198 377 A1 (GELMEDIX INC [US]) 14 April 2022 (2022-04-14) * table 1 * * claims 1-15 * ----- | 1-10 | |
| | | | TECHNICAL FIELDS SEARCHED (IPC) |

The present search report has been drawn up for all claims

1

| Place of search | Date of completion of the search | Examiner |
|---|---|---|
| Munich | 3 August 2023 | Meiser, Wibke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 23 15 7569

This annex lists the patent family members relating to the patent documents cited in the above-mentioned European search report. The members are as contained in the European Patent Office EDP file on
The European Patent Office is in no way liable for these particulars which are merely given for the purpose of information.

03-08-2023

| Patent document cited in search report | | Publication date | Patent family member(s) | | Publication date |
|---|---|---|---|---|---|
| CN 106474049 | A | 08-03-2017 | NONE | | |
| CN 114146226 | A | 08-03-2022 | NONE | | |
| CA 3198377 | A1 | 14-04-2022 | AU 2021356591 | A1 | 08-06-2023 |
| | | | CA 3198377 | A1 | 14-04-2022 |
| | | | EP 4225832 | A1 | 16-08-2023 |
| | | | TW 202229419 | A | 01-08-2022 |
| | | | WO 2022076505 | A1 | 14-04-2022 |

EPO FORM P0459

For more details about this annex : see Official Journal of the European Patent Office, No. 12/82